# EUROPEAN PATENT SPECIFICATION

(11) **EP 0 200 767 B1**
(45) Date of publication and mention of the grant of the patent: **01.06.1994**
(21) Application number: 85905580.8
(22) Date of filing: 31.10.1985
(51) Int. Cl.: G01N 33/52, G01N 33/53, G01N 33/543, G01N 33/546, G01N 33/569

(54) **SANDWICH IMMUNOASSAY OF ANTIGENS INVOLVED IN OPHTHALMIC DISORDERS**
SANDWICH-IMMUNOTESTVERFAHREN FÜR ANTIGENE BEI OPHTHALMISCHEN STÖRUNGEN
ESSAI IMMUNOLOGIQUE A LIAISON DOUBLE D'ANTIGENES IMPLIQUES DANS DES AFFECTIONS DES YEUX

(30) Priority: 31.10.1984 US 667002
(43) Date of publication of application: 12.11.1986
(73) Proprietor: ALCON LABORATORIES, INC., Fort Worth Texas 76101 (US)
(72) Inventor: ROBERTSON, Stella, M., Arlington, TX 76107 (US)
(74) Representative: Kyle, Diana
(86) International application number: US8502146
(87) International publication number: WO8602733

(56) References cited:
- EP-A- 0 017 460
- EP-A- 0 051 096
- EP-A- 0 063 810
- EP-A- 0 113 075
- US-A- 4 376 110
- US-A- 4 391 911
- US-A- 4 427 415
- US-A- 4 430 437
- US-A- 4 430 437
- US-A- 4 514 508
- US-A- 4 535 057
- N, The Journal of Infectious Diseases, Vol. 140, No. 4, issued October 1979, W.B. Davis et al., Ocular Infection with Herpes Simplex Virus Type 1 ...', pages 534-540
- N, Proc. Nat. Acad. Sci USA, Vol. 68, No. 12, issued Dexember 1971, A.M. Brier et al.; 'Inhibition or Enhancement of Immunological Injury of Virus-Infexted Cells', pages 3073-3077
- N. Science, Vol. 219, No. 4585, Issued 11 February 1983, R.C. Nowinski, et al,, 'Monoclonal Antibodies for Diagnosis of Infextious Diseases in Humans', pages 637-644
- N, Journal of Immunological Methods, Vol. 42, No. 1, issued 1981, M.Uotila et al., 'Two-Site Sandwich Enzyme Immunoassay with Monoclonal Antibodies to Human Alpha-Fetoprotein', pages 11-15
- N, Journal of Clinical Microbiology, Vol. 18, No. 2, issued August 1983, R.M. Coleman et al., 'Determination of Herpes Simplex Virus Type-Specific Antibodies by Enzyme-Linked Immunosorbent Assay', pages 287-291

## Description

### BACKGROUND OF THE INVENTION

### 1. Field of the Invention

This invention relates to diagnostic kits for the detection of ophthalmic disorders attributable to antigens. More specifically, this invention relates to diagnostic kits which employ an antibody immunoassay system to detect the presence of antigens in a fluid specimen, such as tears, urine, saliva, blood, spinal fluid, or a solubilized tissue scraping of the affected area.

### 2. Description of Related Art

There is, at present, no quick, inexpensive, and efficient method of detecting many types of disorders attributable to antigens; this is particularly true for ocular disorders, such as Herpes Simplex Virus infections. Two types of methods are currently used to detect Herpes Simplex Virus infections. One of these methods requires that the virus cells be grown in culture, which can take three to seven days, while the other method involves the use of fluorescent microscopy and requires the presence of a trained operator. Neither method allows detection of the virus by the ophthalmologist during the patient's visit to his office. If diagnosed, effective therapy is available for treatment of the viral infection. If the infection is not promptly treated, devastating effects can occur, including severe conjunctivitis, epithelial erosions, and stromal or retinal disease. In fact, Herpes Simplex is a leading cause of blindness due to corneal ulceration and uveitis.

There are a number of diagnostic kits commercially available for testing for the presence of genital Herpes Simplex I and Herpes Simplex II viruses. The kits which use the ELISA system include the kit sold as MA Bioproducts, which measures the amount of antibody present in sera, and a kit sold by Dakopatts which measures the amount of virus. Dakopatts also sells a peroxidase enhanced version which utilizes a cell staining technique. Electro-Nucleonics Labs sells a kit as HSVI FITC, which utilizes a cell staining technique; and Genetic Systems provides a kit which also utilizes a cell staining technique. All of these kits are for use in the detection of genital, rather than ocular herpes.

None of the diagnostic systems currently on the market for measuring ocular infections utilize monoclonal antibody techniques. Immunoassays utilizing monoclonal antibody techniques are described, however, for example, in U.S. Patent No. 4,376,110, assigned to Hybritech, Incorporated. This patent describes immunometric assays known to the prior art as "forward" assays, in which the antibody is bound to a solid phase, and said solid phase is contacted with the sample being tested to extract the antigen from the sample by formation of a binary solid phase antibody-antigen complex. After a suitable incubation period, the solid support is washed to remove the residue of the fluid sample. The solid support is then contacted with a solution containing a known quantity of labeled antibody. The solid support is washed again to remove unreacted labeled antibody. Finally, the support is tested to detect the presence of labeled antibody. This kind of assay is also referred to as a "two side" or "sandwich" assay since the antigen has two antibodies bound to its surface at different locations.

U.S. Patent No. 4,376,110 also describes a second type of assay called a "simultaneous" or "reverse" assay. This type of assay involves a single incubation step in which the sample being tested and the labeled antibody are simultaneously added to the antibody bound to the solid support. After incubation, the solid support is washed to remove the residue of any fluid sample and uncomplexed, labeled antibody.

Both of the above-described techniques were originally developed using so called "polyclonal" antibodies. When an immunogenic substance is introduced into a living body, the body's immune system reacts by generating antibodies to every site it recognizes on the immunogen. A large immunogenic protein molecule may have dozens of sites and a foreign cell may have hundreds. Thus, while each antibody producing cell produces antibodies specific for a single antigenic site, the immune system in total produces large quantities of antibodies specific to hundreds of thousands of different antigens. Therefore, most of the antibodies in a given sample of antisera are not specific for the antigen of interest. Accordingly, the antibodies derived from this antisera, the so called polyclonal antibodies, are very difficult to purify.

U.S. Patent No. 4,376,110 discloses one method of increasing sensitivity of these forward and reverse immunoassays by replacing the polyclonal antibodies with monoclonal antibodies. The production of monoclonal antibodies involves injecting an animal with an immunogen, removing spleen cells from the animal, and fusing these cells with myeloma cells to produce a "hybridoma" which reproduces in vitro. The hybridoma produces antibodies which can be screened to allow isolation of the cells producing the desired antibodies. Cells can then be cloned and the antibodies grown in culture. Immunoassays using such monoclonal antibodies have been shown to have much greater sensitivity to the presence of the antigenic substances than did the assays using the polyclonal antibodies.

EP-A-0063810 relates to devices and kits for immunological analysis the device consisting of a porous solid support containing a pre-selected array of delimited adsorption areas of antigens and/or immunoglobulins. The device is not described as suitable for ophthalmic use. EP-A-0017460 relates to an immunofluorescent test method using stabilized reticulated body derived from C. trachomatis as the antigen in order to detect the formation of an antigen-antibody complex.

### Summary of the Invention

It is one objection of the present invention to develop a diagnostic immunoassay kit which allows simple, rapid detection of the presence of ocular infections caused by Herpes Simplex Virus, Andenovirus, Chlamydia or other bacterial and viral agents.

A further object of the present invention is to develop a diagnostic kit which allows detection of ocular infections, within a short period of time.

Another objection of the present invention is to provide a diagnostic kit which is inexpensive, requires no major equipment, and can be used without special, extensive training.

A further object of this invention is to provide extremely sensitive diagnostic kits employing monoclonal antibody immunoassays.

A still further object of the present invention is to provide diagnostic kits specifically for detecting Herpes Simplex Virus I and II in ocular infections, and to provide a method of detecting ocular Herpes Simplex Virus I and II using these kits.

In satisfaction of these objects the present invention provides a diagnostic kit adapted for detection, in a time period of thirty minutes or less, of ophthalmic disorders attributable to antigens, comprising:
a) a solid substrate on to which are bound monoclonal antibodies complementary to an antigen to be supplied by the ophthalmic specimen to be tested;
b) a first washing solution;
c) a solution containing labeled monoclonal antibodies which are complementary to the antigen to be supplied by the ophthalmic specimen to be tested; and
d) a second washing solution.

The invention further provides a method for the detection, in a time period of thirty minutes or less, of ophthalmic disorders attributable to antigens, comprising:
a) obtaining a solid substrate onto which are bound monoclonal antibodies complementary to a particular antigen;
b) contacting the solid substrate with an ophthalmic fluid specimen containing the antigen, cells infected with the antigen, or non-infected cells;
c) washing the resulting fluid ophthalmic specimen treated with substrate;
d) contacting the washed substrate with a detection probe solution containing labeled monoclonal antibodies complementary to the particular antigen;
e) washing the resulting product; and
f) contacting the substrate with a source of light of sufficient wavelength to allow visual detection of any labeled monoclonal antibodies contained on the substrate.

### Brief Description of the Drawings

Reference is now made to the drawings accompanying the application, wherein
Figure 1 illustrates the assay theory of the diagnostic kit; and
Figure 2 illustrates the assay procedure.

### Detailed Description of the Invention

As indicated above, this invention relates to a diagnostic kit for detecting ocular disorders attributable to antigens which kit makes use of a monoclonal antibody immunoassay system. The antigens which may be detected using the kit of the present invention include, for example: viruses, such as Herpes Simplex Viruses I and II (HSV I and II), Adenovirus, Varicella Zoster virus, Cytomegalovirus, Epstein-Barr virus, Newcastle virus, Measles virus, Coxsackievirus, and Human T Cell Leukemia Virus (HTLV III, also commonly known as the "AIDS" virus); bacteria, such as Chlamydia, Streptococcus, Staphylococcus aureus, Haemophilus, Pseudomonas, Neisseria, Pneumococcus, Moraxella, and Rickettsia; and fungi such as Candida.

The diagnostic kit of this invention is particularly suitable for the detection of ophthalmic infections, such as Herpes Simplex Virus (types I and/or II), Adenovirus and Chlamydia infections. As pointed out above, there is at present no quick, efficient and inexpensive diagnostic procedure for the detection of such pathogens, especially in ocular infections. The diagnostic kit of this invention is designed to obtain rapid positive or negative results from the test and is particularly designed for use in the ophthalmologist's office.

The diagnostic kit of this invention is based on the use of monoclonal antibodies in the detection. As is known, Herpes Simplex Virus Type I (HSV-I) causes a variety of clinical conditions including acute primary infections, chronic infections with continued or intermittent virus shedding, and clinical reactivation. The primary infection with Herpes Simplex Virus-I causes acute gingivostomatitis, rhinitis, keratoconjunctivitis, meningoencephalitis, eczema herpeticum and traumatic herpes, including herpetic whitlow and generalized cutaneous herpes simplex.

In such conditions, follicular conjunctivitis with chemosis, edema of the lids and conjunctival ulcers may be seen. The cornea may be involved in primary or recurrent infections, most of these infections being due to HSV-I. Thus, HSV-I is a leading cause of corneal opacities in the United States. In laboratory animals, an HSV-I infection results in the spread of HSV-I from the sites of the infection along sensory nerves to the central nervous system and, once in the central nervous system, the HSV-I virus can replicate in the brain and kill the host.

It has been reported that the passive transfer of HSV-I specific antibody as late as forty-eight hours post infection can promote recovery from ocular HSV-I infection by inhibiting the spread of the virus within the brain after its entry into the central nervous system. Davis et al., J. Infect. Dis. 140, 534-539 (1979). The mechanism by which antibody inhibits virus spread within the central nervous system is not clear.

Previous studies have shown that virus-specific antibodies can interact with glycoproteins gA/B, gC, and gD, at the sites or surfaces of HSV-I infected cells in vitro to mediate immunocytolysis in the presence of either complement or peripheral blood leukocytes. See Briar et al., PNAS, U.S.A. 68, 3073-3077 (1976). In addition, these glycoproteins, when at the surface of HSV virions, can serve as targets for neutralizing antibody. At present, it is not known which of these glycoproteins or combinations of glycoproteins can interact with antibody in vivo to initiate recovery from ocular HSV-I infection. It is also not clear whether immunocytolysis and virus neutralization mediated by each of these glycoproteins in vitro contribute to the clearance of HSV-I in vivo.

The present invention, therefore, provides a diagnostic kit designed to provide early detection of a serious ocular disease which can lead to complications and even blindness, as well as detecting many other types of ocular and nonocular disorders. The diagnostic kit of the present invention is based on the idea of using immunologic technology in ophthalmic diagnosis.

The diagnostic kit requires the use of monoclonal antibodies (MAB) hooked to a solid substrate. A fluid specimen, such as a teardrop or fluid containing a solubilized tissue scraping from the affected area, is then applied to the solid substrate and incubated long enough to allow the dissolved antigens contained in the fluid specimen to complex with the immobilized, antigen-specific, monoclonal antibodies present on the substrate. Following this incubation, the solid substrate is washed with a first washing solution. The solid substrate is then incubated with a solution containing labeled monoclonal antibodies which are specific for the immobilized antigen present on the solid substrate; this incubation must continue for a sufficient time to allow the labeled monoclonal antibodies to combine with the immobilized antigen. The solid support is then washed one or more times with a neutral wash solution in order to remove any unbound, labeled monoclonal antibodies, since such unbound, labeled antibodies would give a false, high reading.

The above-described diagnostic kit provides an assay which can be highly specific for the antigen by the use of monoclonal antibodies. The assay is faster than the use of a culture or ELISA and the assay does not require sophisticated equipment such as that required by the special ELISA readers or fluorescent microscopes. Further, the diagnostic kits and assays ace designed specifically for the needs of the practicing ophthalmologist.

A basic aspect of the invention is to provide a support or substrate on which the monoclonal antibodies may be immobilized. The support can be any known substrate or matrix to which proteins can be permanently applied. Suitable solid supports include paper, nylon membranes or fibers, latex beads, sugar beads, glass beads, agarose beads, albumin beads, glass slides, plastic, acetate, polyvinyl chloride, or any other matrix onto which proteins can be permanently applied. The matrix pads described in U.S. Patent No. 3,888,629, represent a preferred type of substrate. The monoclonal antibodies are bound to the support using standard protein binding techniques. This step results in a complex consisting of the solid support and either immobilized monoclonal antibodies or immobilized antigen.

The fluid specimen is preferably tears. Alternatively, sterile fluid containing solubilized tissue scrapings from the affected area or area juxtaposed to the affected area can be used.

Possible labels for the monoclonal antibodies include enzymes such as peroxidase, phosphatase and oxidase, and dyes or dye complexes such as fluorescein isothiocyanate (FITC), FITC-Avidin/Biotin or FITC-Staph A, or other enzymes or dyes which would allow visual detection when contacted with the proper wavelength of light. The solution containing the labeled monoclonal antibodies is incubated with the solid support complex for a sufficient time to allow the labeled monoclonal antibodies to combine with the immobilized antigen.

Various means for detecting the presence of labeled monoclonal antibodies on the solid substrate may be utilized. If FITC was used as the monoclonal antibody label, its presence can be detected using ultraviolet light. This ultraviolet light can be supplied by a slit lamp to excite the dye; a lamp of this type is a common fixture in an ophthalmologist's office. Other detection methods can also be used, such as identification of a positive color, precipitation spot or agglutination.

There are a number of advantages to the use of the above-described diagnostic test kit. The kit will be self-contained and sterile, will have a minimum shelf life of three to six months, will provide a YES/NO answer for the presence of a specific antigen (e.g., an ophthalmic virus) in a relatively short time (i.e., on the order of about thirty minutes or less), and can be used without any special equipment or training. In a preferred embodiment, the kits will contain the following components:
1) paper, plastic, or membrane strips impregnated with monoclonal antibodies which are complementary to the subject antigen,
2) neutral, sterile, wash solutions, each packaged in separate bottles; and
3) a vial containing a detection probe solution.
The probe solution contains labeled monoclonal antibodies. The user of the kit (e.g., an ophthalmologist) would normally supply the light source in the form of a slit lamp or other source of ultraviolet or shortwave light.

There are a number of commercially available monoclonal antibodies which may find use in the diagnostic kit of the present invention. For example, anti-Herpes Simplex Virus I monoclonal antibodies identified as 9150 IgM, 9151 IgG1, and 9152 IgG1, are available from Biotech Products. Bethesda Research Laboratories have available monoclonal antibodies effective against Herpes Simplex Virus I and II, which antibodies are identified as 35004 IgG2a Anti gp D. Cappel Laboratories produces an anti-Herpes Simplex Virus I antibody identified as 0238-2967 IgG1 Anti p 40-45 Nucleocapsid.

Anti-Adenovirus antibodies which may be utilized in the kit of the present invention include those of the type described in U.S. Patent No. 4,487,829, assigned to Massachusetts Institute of Technology, particularly the antibody identified as 2Hx-2. These antibodies are further described in the following scientific article: Sharp et al, "Adenovirus Hexon Monoclonal Antibody that is Group Specific and Potentially Useful as a Diagnostic Reagent," Journal of Clinical Microbiology, Vol. 17, pages 360-364 (1983) and U.S. Patent No. 4,487,829. Additional anti-Adenovirus antibodies suitable for use in the kit of the present invention are described in an article by Heirholzer et al., titled "The Application of a Solid-Phase Immunofluorometric Assay to the Selection of Monoclonal Antibody Specific for the Adenovirus Group-Reactive Hexon Antigen," Archives of Virology, Vol. 80, pages 1-10 (1984). Of the antibodies described by Heirholzer et al., those identified as 2/6 and 20/11 are believed to be especially suitable for use in the present invention.

The anti-Chlamydia antibodies which may be utilized in the present invention include those of the type described in the following scientific articles: Cho-Chou Kuo et al., "Sensitivity of Immunofluorescence with Monoclonal Antibodies for Detection of Chlamydia Trachomatis Inclusions in Cell Culture", Journal of Clinical Microbiology, Vol. 16, pages 4-7 (1982); and Cho-Chou Kuo et al., "Monoclonal Antibodies to Chlamydia Trachomatis: Antibody Specificities and Antigen Characterization", The Journal of Immunology, Vol. 128, (1982); more particularly, antibodies of the type produced by the hybridomas designated as 2Cl and l H 8. Such antibodies are group specific for chlamydia trachmonatis.

A preferred embodiment of this invention utilizes monoclonal antibodies produced by hybridomas having the following designations and specificities:

| Designation | Specificity | |
|---|---|---|
| 9D8.H | Anti-gpC | HSV-I |
| 9F8.A | Anti-gpC | HSV-I |
| 7B2B.D | Anti-gpA/B | HSV-I and II |
| 11B3A.G | Anti-gpD | HSV-I and II |
| 4E3 | Anti-gpD | HSV-I and II |
| 4G4 | Anti-gpD | HSV-I and II |

These hybridomas were developed at Alcon Laboratories, Inc. The hybridomas were obtained from the fusion of the myeloma Sp2/O and appropriately sensitized lymphocytes from the spleen of a Balb/C mouse immunized with HSV-I. These hybridomas have been deposited at the American Type Culture collection (ATCC), Rockville, Maryland.

Another preferred embodiment of the present invention uses hybridoma secreted anti-Herpes Simplex Virus-I monoclonal antibodies. The hybridomas from which the antibodies are secreted are made by fusion of a myeloma cell line and appropriately sensitized lymphocytes, wherein said lymphocytes are derived from a SJL/J mouse. The hybridomas from which the monoclonal antibodies are secreted are identified as hybridomas D8AB, G8C, H7E and F3AB. These hybridomas were deposited at the American Type Culture Collection (ATCC), Rockville, Maryland, and assigned accession numbers as follows:

| | |
|---|---|
| D8AB | HB8364 |
| G8C | HB8367 |
| H7E | HB8365 |
| F3AB | HB8366 |

Various other types of antibodies may also be utilized in the diagnostic kit of the present invention, as will be appreciated by those skilled in the art.

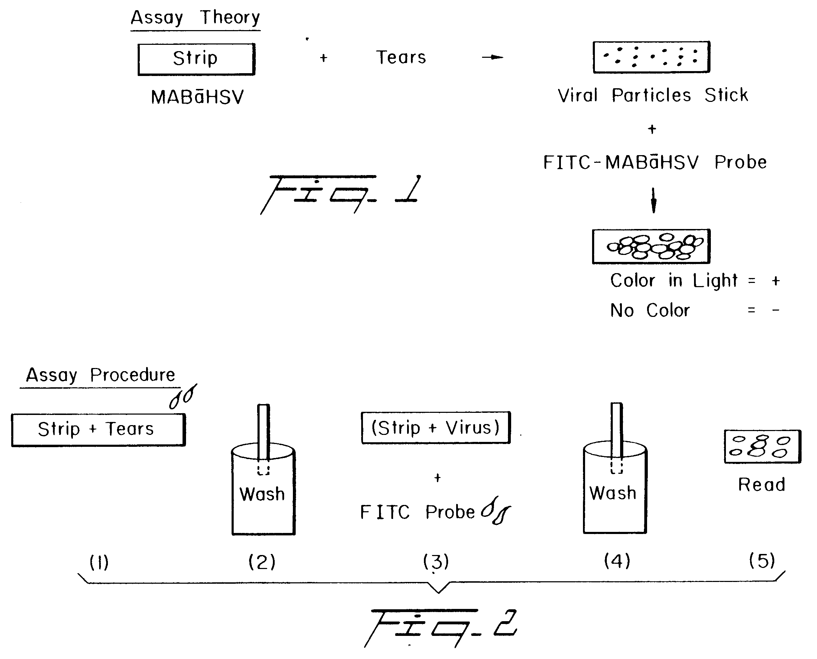

Reference is now made to the drawings accompanying the application wherein it will be seen that Figure 1 describes the assay theory of the invention and Figure 2 describes the assay procedure. In Figure 1 it will be seen that the strip referred to is, for example, a paper strip which has been impregnated with anti-Herpes Simplex Virus I and II monoclonal antibodies. On contact of the body fluid such as tears with the paper strip there results the paper strip to which the viral particles are attached as shown in Figure 1. The impregnated paper in which the viral particles are present is then incubated with the probe which contains FITC or another dye to allow visual detection. After washing as described above, a positive test can then be detected from the light source.

Figure 2 provides the assay procedure wherein it will be seen that the first step involves the paper strip (1) containing anti-Herpes Simplex Virus-I monoclonal antibodies impregnated with the tears. This paper strip is then preferably washed as shown at (2), after which the washed strip containing the virus is incubated at (3) with the probe containing the dye such as FITC dye. This step is followed by a further wash (4) and finally the light source is used for detection at (5). It can therefore be seen that the assay theory and the assay procedure of the present invention are quick and convenient.

The solid substrate can take a number of alternative embodiments.

For example, a paper strip may be prepared having a localized area or areas of immobilized monoclonal antibodies present thereon. The paper strip and antibodies present thereon can be placed in a test tube which contains a reservoir of the sample liquid at the bottom. The paper strip would then act as a wick, the sample solution traveling up the strip and concentrating at the localized area(s) of monoclonal antibodies. This method has the advantage of concentrating antigen contained in the sample in a smaller area, thereby rendering a much smaller concentration of antigen detectable. Alternatively, the same results could be reached with a paper wick using layered dry chemistry technology. The paper wick may also be attached to the end of an applicator stick for greater ease in use.

In another embodiment, a capillary tube may be prepared containing a solid phase matrix onto which the monoclonal antibodies are immobilized. The matrix is immersed in the sample fluid reservoir at the bottom of the tube, so that the sample fluid flows up the tube over the matrix. The matrix can then be removed from the solution reservoir. A syringe containing water solution and/or monoclonal antibody probe may be attached to the top of the capillary tube. The solution can then be introduced into the top of the tube, and allowed to flow through the capillary. Detection is accomplished by shining a light on the tube.

In still another embodiment, the solid substrate may comprise a capsule, such as an Ocusert™-like capsule, attached to the end of a stick. The outer surface of the capsule is interrupted by pores large enough to allow protein to enter. The monoclonal antibody is immobilized on the inner core matrix. It is this inner core matrix to which the antigen would become bound. This capsule can be placed in the cul-de-sac of the eye. The capsule can then be removed, dipped in a wash solution, and dipped in a cleaning solution to clear material from the pores. The capsule may then be dipped in the monoclonal antibody probe solution and allowed to develop. A light source can then be applied to the capsule for detection.

The diagnostic kit and method of the present invention are further illustrated by the example which follows.

### EXAMPLE

A diagnostic kit in accordance with the present invention may be prepared as follows. First, a test strip is prepared by attaching and immobilizing monoclonal antibodies on a strip of nitrocellulose (Biorad) paper or nylon membrane (Pall) having dimensions of approximately 30 x 5 mm. The antibodies are attached to the surface of the test strip in areas of discrete spots of approximately 1 to 3 mm in diameter. The test strip may be then mounted on a matrix substance, such as filter paper, which allows absorption and wicking of fluids. Next, areas of the test strip not containing the monoclonal antibodies are blocked by coating the strip with a gelatin - containing buffered saline solution, thereby isolating and immobilizing the discrete spots of monoclonal antibodies.

The diagnostic kit is completed by preparing first and second washing solution vials, each of said vials containing 1 to 2 ml of a washing solution consisting of a buffered saline solution, tween 20 and an inert protein filler; and preparing one vial containing a detection probe consisting of a mixture of monoclonal antibodies which are complexed with biotin and have Fluorescein labelled avidin attached thereto as an amplification molecule.

The above-described diagnostic kit may be utilized for detecting ocular Herpes Simplex Virus I and II, according to the method of the present invention, as follows. First, a 1 µL to 30 µL sample containing virus, virally infected cells and/or non-infected cells is applied to the test strip for 10 minutes at room temperature. Excess sample is removed by immersing the test strip in the first washing solution vial and soaking the strip with intermittent agitation for 5 minutes. The test strip is then immersed in the vial containing the detection probe for 10 minutes at room temperature with intermittent agitation. Excess probe is removed by immersing the strip in the second washing solution vial with agitation for 5 minutes. The strip is then air dried. After drying, the strip is examined under a shortwave light source. A positive sample is visualized by the bright green appearance of the discrete spots when a shortwave light source is focused on the test strip.

## Claims

1. A diagnostic kit adapted for detection, in a time period of thirty minutes or less, of ophthalmic disorders attributable to antigens, comprising:
a) a solid substrate onto which are bound monoclonal antibodies complementary to an antigen to be supplied by the ophthalmic specimen to be tested;
b) a first washing solution;
c) a solution containing labeled monoclonal antibodies which are complementary to the antigen to be supplied by the ophthalmic specimen to be tested; and
d) a second washing solution.

2. A diagnostic kit according to claim 1, wherein the monoclonal antibodies are selected from the group consisting of anti-Herpes Simplex Virus monoclonal antibodies selected from anti-Herpes Simplex Virus I monoclonal antibodies, anti-Herpes Simplex Virus II monoclonal antibodies, and anti-Herpes Simplex Virus I and II monoclonal antibodies; anti-Chlamydia monoclonal antibodies; anti-Adenovirus monoclonal antibodies; and anti-Human T Cell Leukemia Virus monoclonal antibodies.

3. A diagnostic kit according to claim 2, wherein the monoclonal antibodies are labeled with a dye or enzyme which will allow visual detection when contacted by a light source of proper wave length.

4. A diagnostic kit according to claim 3, wherein the solid substrate onto which the monoclonal antibodies are bound is selected from the group consisting of paper, nylon fibers, nylon membranes, latex beads, sugar beads, albumin beads, agarose beads, glass beads, glass slides, plastic, acetate, and polyvinyl chloride.

5. A diagnostic kit according to claim 3, wherein the monoclonal antibodies comprise anti-Herpes Simplex Virus monoclonal antibodies secreted by a hybridoma, said hybridoma having been made by the fusion of a myeloma cell line and appropriately sensitized lymphocytes, said lymphocytes being derived from a Balb/C mouse.

6. A diagnostic kit according to claim 5, wherein the solid substrate onto which the monoclonal antibodies are bound is selected from the group consisting of nylon membranes and paper, and the wash solutions are neutral sterile solutions of water packaged in separate bottles and the solution containing the labeled monoclonal antibodies is a container provided with a dropper for sterile use.

7. A method for the detection, in a time period of thirty minutes or less, of ophthalmic disorders attributable to antigens, comprising:
a) obtaining a solid substrate onto which are bound monoclonal antibodies complementary to a particular antigen;
b) contacting the solid substrate with an ophthalmic fluid specimen containing the antigen, cells infected with the antigen, or non-infected cells;
c) washing the resulting fluid ophthalmic specimen treated substrate;
d) contacting the washed substrate with a detection probe solution containing labeled monoclonal antibodies complementary to the particular antigen;
e) washing the resulting product; and
f) contacting the substrate with a source of light of sufficient wavelength to allow visual detection of any labeled monoclonal antibodies contained on the substrate.

8. A method according to claim 7, wherein the monoclonal antibodies are selected from the group consisting of anti-Herpes Simplex Virus monoclonal antibodies selected from anti-Herpes Simplex Virus I monoclonal antibodies, anti-Herpes Simplex Virus II monoclonal antibodies, and anti-Herpes Simplex Virus I and II monoclonal antibodies; anti-Chlamydia monoclonal antibodies; anti-Adenovirus monoclonal antibodies; and anti-Human T cell Leukemia Virus monoclonal antibodies.

9. A method according to claim 8, wherein the monoclonal antibodies comprise anti-Herpes Simplex Virus monoclonal antibodies secreted by a hydridoma, said hybridoma having been made by the fusion of a myeloma cell line and appropriately sensitized lymphocytes, said lymphocytes being derived from a Balb/C mouse.

10. Use of labeled anti-Herpes Simplex Virus monoclonal antibodies in the manufacture of a composition for the detection, in a time period of thirty minutes or less, of ophthalmic disorders attributable to antigens by the detection method according to claim 7, said monoclonal antibodies having been secreted by a hybridoma made by the fusion of a myeloma cell line and appropriately sensitized lymphocytes, said lymphocytes being derived from a Balb/C mouse, said monoclonal antibodies being selected from the group consisting of anti-Herpes Simplex Virus I monoclonal antibodies, anti-Herpes Simplex Virus II monoclonal antibodies, anti-Herpes Simplex Virus I and II monoclonal antibodies.

11. Use of labeled anti-Herpes Simplex Virus monoclonal antibodies according to claim 10 wherein said antibodies are anti-Herpes Simplex Virus I and II monoclonal antibodies.

## Patentansprüche

1. Diagnostisches Kit, das an den Nachweis von Antigen zuzuschreibenden ophthalmischen Erkrankungen in einer Zeitspanne von 30 Minuten oder weniger angepaßt ist, umfassend:
(a) ein festes Substrat, an welches monoklonale Antikörper gebunden sind, die komplementär zu einem Antigen sind, das mit der zu testenden ophthalmischen Probe bereitgestellt wird;
(b) eine erste Waschlösung;
(c) eine Lösung, die markierte monoklonale Antikörper enthält, welche komplementär zu dem Antigen sind, das mit der zu testenden ophthalmischen Probe bereitgestellt wird; und
(d) eine zweite Waschlösung.

2. Diagnostisches Kit gemäß Anspruch 1, worin die monoklonalen Antikörper ausgewählt sind aus der Gruppe bestehendend aus monoklonalen Anti-Herpes-Simplex-Virus Antikörpern, ausgewählt aus monoklonalen Anti-Herpes-Simplex-Virus I Antikörpern, monoklonalen Anti-Herpes-Simplex-Virus II Antikörpern und monoklonalen Anti-Herpes-Simplex-Virus I und II Antikörpern; monoklonalen Anti-Chlamydien Antikörpern; monoklonalen Anti-Adeno-Virus Antikörpern; und monoklonalen Anti-Human T Zell Leukämie-Virus Antikörpern.

3. Diagnostisches Kit gemäß Anspruch 2, worin die monoklonalen Antikörper mit einem Farbstoff oder Enzym markiert sind, welches, falls mit einer Lichtquelle geeigneter Wellenlänge in Berührung gebracht, den visuellen Nachweis ermöglicht.

4. Diagnostisches Kit gemäß Anspruch 3, worin das feste Substrat, an welches die monoklonalen Antikörper gebunden sind, aus der Gruppe bestehend aus Papier, Nylonfasern, Nylonmembranen, Latexperlen, Zuckerperlen, Albuminperlen, Agaroseperlen, Glasperlen, Glasplättchen, Plastik, Acetaten und Polyvinylchlorid ausgewählt ist.

5. Diagnostisches Kit gemäß Anspruch 3, worin die monoklonalen Antikörper monoklonale Anti-Herpes-Simplex-Virus Antikörper umfassen, die von einem Hybridom abgeschieden werden, wobei das Hybridom durch Fusion einer Myeloma Zellinie und geeignet sensibilisierten Lymphocyten, die von einer Balb/C Maus herrühren, hergestellt worden ist.

6. Diagnostisches Kit gemäß Anspruch 5, worin das feste Substrat, an welches die monoklonalen Antikörper gebunden sind, aus der Gruppe bestehend aus Nylonmembranen und Papier ausgewählt ist, die Waschlösungen neutrale sterile Lösungen von Wasser, die in getrennten Flaschen verpackt sind, sind und die die markierten monoklonalen Antikörper enthaltende Lösung ein Container ist, der zur sterilen Verwendung mit einem Tropfenzähler versehen ist.

7. Verfahren für das Nachweisen von Antigenen zuzuschreibenden ophthalmischen Erkrankungen in einer Zeitspanne von 30 Minuten oder weniger umfassend:
a) Bereitstellen eines festen Substrates, an welches monoklonale Antikörper, die komplementär zu einem bestimmten Antigen sind, gebunden sind;
b) In Berührung bringen des festen Substrates mit einer das Antigen enthaltenden ophthalmischen Flüssigkeitsprobe, mit dem Antigen infizierten Zellen oder nicht infizierten Zellen;
c) Waschen des erhaltenen mit ophthalmischer Flüssigprobe behandelten Substrates;
d) In Berührung bringen des gewaschenen Substrates mit einer Nachweissondenlösung, die markierte monoklonale Antikörper, die komplementär zu dem betreffenden Antigen sind, enthält;
e) Waschen des erhaltenen Produktes; und
f) In Berührung bringen des Substrates mit einer Lichtquelle von ausreichender Wellenlänge, um den visuellen Nachweis jedes an dem Substrat enthaltenden markierten monoklonalen Antikörpers zu erlauben.

8. Verfahren gemäß Anspruch 7, worin die monoklonalen Antikörper ausgewählt sind aus der Gruppe bestehend aus monoklonalen Anti-Herpes-Simplex-Virus Antikörpern, monoklonalen Anti-Herpes-Simplex-Virus I Antikörpern, monoklonalen Anti-Herpes-Simplex-Virus II Antikörpern und monoklonalen Anti-Herpes-Simplex-Virus I und II Antikörpern; monoklonalen Anti-Chlamydien Antikörpern; monoklonalen Anti-Adeno-Virus Antikörpern; und monoklonalen Anti-Human T Zell Leukämie-Virus Antikörpern.

9. Verfahren gemäß Anspruch 8, worin die monoklonalen Antikörper monoklonale Anti-Herpes-Simplex-Virus Antikörper umfassen, die von einem Hybridom abgeschieden werden, wobei das Hybridom durch Fusion einer Myeloma Zellinie und geeignet sensibilisierten Lymphocyten, die von einer Balb/C Maus herrühren, hergestellt worden ist.

10. Verwendung von markierten monoklonalen Anti-Herpes-Simplex-Virus Antikörpern für die Herstellung einer Zusammensetzung für den Nachweis von Antigenen zuzuschreibenden ophthalmischen Erkrankungen in einer Zeitspanne von 30 Minuten oder weniger durch das Nachweisverfahren gemäß Anspruch 7, wobei die monoklonalen Antikörper von einem Hybridom abgeschieden werden, das durch die Fusion einer Myeloma Zellinie und geeigneten sensibilisierten Lymphozyten, wobei die Lymphozyten von einer Balb/C Maus herrühren, hergestellt sind und wobei die monoklonalen Antikörper aus der Gruppe bestehend aus monoklonalen Anti-Herpes-Simplex-Virus I Antikörpern, monoklonalen Anti-Herpes-Simplex-Virus II Antikörpern, monoklonalen Anti-Herpes-Simplex-Virus I und II Antikörpern ausgewählt sind.

11. Verwendung von markierten monoklonalen Anti-Herpes-Simplex-Virus Antikörpern gemäß Anspruch 10, worin die Antikörper monoklonale Anti-Herpes-Simplex-Virus I und II Antikörper sind.

## Revendications

1. Trousse de diagnostic adaptée pour la détection, en uns durée égale ou inférieure à trente minutes, de maladies ophtalmiques attribuables à des antigènes, comprenant:
a) un substrat solide sur lequel sont liés des anticorps monoclonaux complémentaires d'un antigène à fournir par l'échantillon ophtalmique que l'on teste;
b) une première solution de lavage;
c) une solution contenant des anticorps monoclonaux marqués qui sont complémentaires de l'antigène à fournir par l'échantillon ophtalmique à tester; et
d) une seconde solution de lavage.

2. Trousse de diagnostic selon la revendication 1, dans laquelle les anticorps monoclonaux sont choisis dans le groupe constitué par les anticorps monoclonaux anti-virus d'Herpes simplex choisis parmi les anticorps monoclonaux anti-virus d'Herpes simplex I les anticorps monoclonaux anti-virus d'Herpes simplex II, et les anticorps monoclonaux anti-virus d'Herpes simplex I et II; les anticorps monoclonaux anti-chlamydiens; les anticorps monoclonaux anti-adénovirus; et les anticorps monoclonaux anti-virus de leucémie des cellules T humaines.

3. Trousse de diagnostic selon la revendication 2, dans laquelle les anticorps monoclonaux sont marqués avec un colorant ou une enzyme qui permet la détection visuelle lorsqu'on les met en contact avec une source de lumière de longueur d'onde appropriée.

4. Trousse de diagnostic selon la revendication 3, dans laquelle le substrat solide sur lequel les anticorps monoclonaux sont liés est choisi dans le groupe constitué par le papier, les fibres de nylon, les membranes de nylon, les perles de latex, les perles de sucre, les perles d'albumine, les perles d'agarose, les perles de verre, les lames de verre, les matières plastiques, l'acétate et le chlorure de polyvinyle.

5. Trousse de diagnostic selon la revendication 3, dans laquelle les anticorps monoclonaux comprennent des anticorps monoclonaux anti-virus d'Herpes simplex sécrétés par un hybridome, ledit hybridome ayant été préparé par fusion d'une lignée cellulaire de myélome et de lymphocytes sensibilisés de manière appropriée, lesdits lymphocytes étant dérivés d'une souris Balb/C.

6. Trousse de diagnostic selon la revendication 5, dans laquelle le substrat solide sur lequel les anticorps monoclonaux sont liés est choisi dans le groupe constitué par les membranes de nylon et le papier, et les solutions de lavage sont des solutions stériles neutres d'eau conditionnées dans des flacons séparés et la solution contenant les anticorps monoclonaux marqués est un récipient muni d'un flacon compte-gouttes pour utilisation stérile.

7. Procédé de détection, dans une durée égale ou inférieure à trente minutes, de désordres ophtalmiques attribuables à des antigènes, dans lequel:
a) on obtient un substrat solide sur lequel sont liés des anticorps monoclonaux complémentaires d'un antigène particulier;
b) on met en contact le substrat solide avec un échantillon de fluide ophtalmique contenant l'antigène, dos cellules infectées par l'antigène, oudes cellules non infectées;
c) on lave le substrat traité avec l'échantillon de fluide ophtalmique résultant;
d) on met en contact le substrat lavé avec une solution de sonde de détection contenant des anticorps monoclonaux marqués complémentaires de l'antigène particulier;
e) on lave le produit résultant; et
f) on met on contact le substrat avec une source de lumière de longueur d'onde suffisante pour permettre la détection à l'oeil nu d'anticorps monoclonaux marqués éventuels contenus sur le substrat.

8. Procédé selon la revendication 7, dans lequel les anticorps monoclonaux sont choisie dans le groupe constitué par les anticorps monoclonaux anti-virus d'Herpes simplex choisis parmi les anticorps monoclonaux anti-virus d'Herpes simplex I, les anticorps monoclonaux anti-virus d'Herpes simplex II et les anticorps monoclonaux anti-virus d'Herpes simplex I et II; les anticorps monoclonaux antichlamydiens; les anticorps monoclonaux anti-adénovirus; et les anticorps monoclonaux anti-virus de leucémie des cellules T humaines.

9. Procédé selon la revendication 8, dans lequel les anticorps monoclonaux comprennent des anticorps monoclonaux anti-virus d'Herpes simplex sécrétés par un hybridome, ledit hybridome ayant été contitué par la fusion d'une lignée cellulaire de myélome et de lymphocytes sensibilisés de façon appropriée, lesdits lymphocytes étant dérivés d'une souris Balb/C.

10. Application d'anticorps monoclonaux anti-virus d'Herpes simplex marqués à la préparation d'une composition pour la détection, dans une durée égale ou inférieure à trente minutes, de désordres ophtalmiques attribuables à des antigènes par le procédé de détection selon la revendication 7, lesdits anticorps ayant été sécrétés par un hybridome constitué par la fusion d'une lignée cellulaire de myélome et de lymphocytes sensibilisés de façon appropriée, lesdits lymphocytes étant dérivés d'une souris Balb/C, lesdits anticorps monoclonaux étant choisis dans le groupe constitué par les anticorps monoclonaux anti-virus d'Herpes simplex I, les anticorps monoclonaux anti-virus d'Herpes simplex II et les anticorps monoclonaux anti-virus df'Herpes simplex I et II.

11. Application d'anticorps monoclonaux anti-virus d'Herpes simplex marqués selon la revendication 10, lesdits anticorps étant des anticorps monoclonaux anti-virus d'Herpes simplex I et II.
